# EUROPEAN PATENT SPECIFICATION

(11) **EP 1 300 473 B1**
(45) Date of publication and mention of the grant of the patent: **10.09.2008**
(21) Application number: 01947806.4
(22) Date of filing: 05.07.2001
(51) Int. Cl.: C12Q 1/68, C12Q 1/34, C12Q 1/25, C12N 15/11

(54) **METHOD OF DETECTING NUCLEOTIDE POLYMORPHISM**
METHODE ZUM NACHWEIS VON NUKLEINSÄUREPOLYMORPHISMEN
PROCEDE DE DETECTION DE POLYMORPHISMES NUCLEOTIDIQUES

(30) Priority: 05.07.2000 JP 2000203508
(43) Date of publication of application: 09.04.2003
(73) Proprietor: Toyo Boseki Kabushiki Kaisha, Osaka 530-0004 (JP)
(72) Inventor: TAKARADA, Yutaka, 1-1, Katata 2-chome Otsu-shi, Shiga 520-0243 (JP); AONO, Toshiya, 1-1, Katata 2-chome Otsu-shi, Shiga 520-0243 (JP); SEGAWA, Masaya, 1-1, Katata 2-chome Otsu-shi, Shiga 520-0243 (JP); YOSHIGA, Satoko, 1-1, Katata 2-chome Otsu-shi, Shiga 520-0243 (JP)
(74) Representative: Vossius & Partner
(86) International application number: PCT/JP2001/005792
(87) International publication number: WO 2002/002815

(56) References cited:
- WO-A1-95/21271
- WO-A1-96/31622
- WO-A1-97/45559
- WO-A1-97/46701
- WO-A2-96/06190
- US-A- 5 728 526
- US-A- 6 027 889

### SEQUENCE LISTING

<110> TOYO BOSEKI KABUSHIKI KAISHA
<120> METHOD OF DETECTING NUCLEOTIDE POLYMORPHISM
<130> P01-63
<160> 7
<170> Patentln version 3.1
<210> 1
   <211> 20
   <212> DNA
   <213> Artificial Sequence
<220>
   <223> primer
<400> 1
   tcgggctggg aagatcgagc 20
<210> 2
   <211> 20
   <212> DNA
   <213> Artificial Sequence
<220>
   <223> primer
<400> 2
   ctctgcccct tctcctgcgc 20
<210> 3
   <211> 24
   <212> DNA
   <213> Artificial Sequence
<220>
   <223> oligonucleotide
<400> 3
   tggagaaagg gcctcctctc ttta 24
<210> 4
   <211> 24
   <212> DNA
   <213> Artificial Sequence
<220>
   <223> oligonucleotide
<400> 4
   tggagaaagg gcctcctctc tttt 24
<210> 5
   <211> 19
   <212> DNA
   <213> Artificial Sequence
<220>
   <223> oligonucleotide
<400> 5
   ggaagatggg gacccggca 19
<210> 6
   <211> 20
   <212> DNA
   <213> Artificial Sequence
<220>
   <223> probe
<400> 6
   cccatcttca aaagagagga 20
<210> 7
   <211> 20
   <212> DNA
   <213> Artificial Sequence
<220>
   <223> probe
<400> 7
   cccatcttct aaagagagga 20

## Description

### TECHNICAL FIELD

The present invention relates to a method for analyzing nucleic acid sequences. Specifically, the present invention relates to a nucleic acid sequence analyzing method by which nucleotide polymorphisms contained in specific nucleic acid sequences can be detected. The nucleic acid sequence analyzing method of the present invention is useful in genetic engineering, molecular biology, and related industrial fields.

### BACKGROUND OF THE INVENTION

In the present invention, a nucleotide polymorphism is a genotype having a nucleotide sequence different from that of the wild type. In drug metabolism, polymorphic genes are key factors responsible for variances between individuals in side effects and drug treatment failure. Nucleotide polymorphisms are also known to cause individual variations in the basal metabolism and the like, which are known as constitution. In addition, they serve as genetic labels for various diseases. Consequently, the analysis of such mutation is clinically important, and routine phenotype classification is particularly recommended for clinical studies involving psychiatric patients and suicidal subjects (Gram and Brsen, European Consensus Conference on Pharmacogenetics, Commission of the European Communities, Luxembourg, 1990, pp. 87-96; Balant et al., Eur. J. Clin. Pharmacol. Vol. 36, pp. 551-554 (1989)). For such reasons, there is a demand for methods of analyzing nucleic acid sequences for detecting the various genotypes after identifying the responsible polymorphic genes.

Known techniques for nucleic acid sequence analysis include, for example, the nucleic acid sequencing method (sequencing method). The sequencing method can detect and identify nucleotide polymorphisms contained in nucleic acid sequences, but considerable time and effort are required for template preparation, polymerase reactions, polyacrylamide gel electrophoresis, nucleic acid sequence analysis, etc. Automatic sequencers have simplified this process in recent years; however, the method has a problem of requiring expensive equipment.

As an alternative method, southern hybridization (Masami MURAMATSU Labo. manual gene engineering revised version, MARUZEN CO., LTD. pp. 70-75) can be cited. Employing this method makes it possible to identify a domain of DNA having a nucleotide sequence complementary to that of a labeled DNA probe. In other words, in southern hybridization, a nucleic acid fragment is subjected to electrophoresis on a flat plate of agarose gel or polyacrylamide gel, separated according to the size (length) of the fragment, and denatured into a single chain nucleic acid. Then, a membrane of nitrocellulose, nylon, or the like is attached to the flat plate. The nucleic acid fragment is transferred with its exact electrophoretic pattern intact, and immobilized. Thereafter, a hybrid is formed by the transferred nucleic acid fragment and a nucleotide polymorphism specific DNA probe that is labeled with an RI (a radioactive isotope) or the like, and the nucleic acid fragments on the membrane that are complementary to the probe are detected using autoradiography, etc.

Southern hybridization makes it possible to determine the electrophoretic position and molecular weight of the target nucleic acid fragment. However, the method has certain drawbacks, i.e., prompt analysis is prevented by the time-consuming electrophoresis, autoradiography operation, etc., and, identification of similar sequences for detecting the presence of nucleotide polymorphisms becomes difficult, unless the specificity of the probe is exact, since it performs detection only by determining whether or not the nucleic acid hybridizes with a nucleotide polymorphism specific DNA probe.

In recent years, methods using ligase (Japanese Examined Patent Publication No. 1994-44880 and Patent No. 2622255) and other methods using nuclease (Japanese Unexamined Patent Publication No. 1990-20298 and Japanese Unexamined Patent Publication No.1991-43098) have become known for identifying nucleic acid sequences using enzymes.

Another example for detecting nucleic acid sequence differences is using coupled ligase detection and polymerase chain reaction (US 6,027,889).
US 5,728,526 describes a method for analyzing a nucleotide sequence which uses a polynucleotide fixed on a solid support and is capable of forming a product containing a detectable label as well as kits for conducting the method.
WO 97/46701, WO 96/31622 and WO 95/21271 describe methods for detecting nucleotide polymorphisms using a DNA polymerase and a ligase.
An object of the present invention is to provide a nucleic acid sequence analyzing method which can readily detect the type of nucleotide polymorphism of a specific nucleotide-polymorphism-containing nucleic acid sequence in a sample.

### BRIEF DESCRIPTION OF THE DRAWINGS

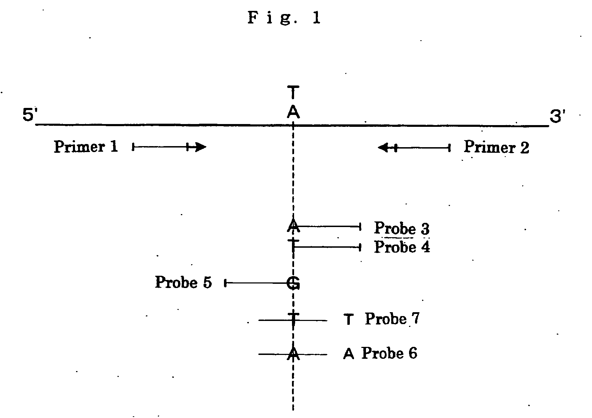

Fig. 1 shows the positions of the oligonucleotides used in the Examples of the present invention.

### DISCLOSURE OF THE INVENTION

In view of the above-mentioned problems, the present inventors conducted extensive research and found that nucleotide polymorphisms can be readily detected by using ligase activity and nuclease activity sequentially and/or simultaneously.

Specifically, the inventors found that nucleotide polymorphisms can be detected by hybridizing
(1) a first wild-type oligonucleotide having a sequence (bases) complementary to wild-type bases in a nucleotide polymorphism site, and a second oligonucleotide adjacently hybridized with the first wild-type oligonucleotide and having a nucleotide sequence not complementary to the wild-type bases in the nucleotide polymorphism site; and
(2) one or more first polymorphism oligonucleotides having a sequence (bases) complementary to polymorphism bases in a polymorphism site, and a second oligonucleotide adjacently hybridized with the first polymorphism oligonucleotide and having a nucleotide sequence not complementary to polymorphism bases in the nucleotide polymorphism site, both (1) and (2) being complementary to one strand of a chromosome or nucleic acid fragment (hereinafter a nucleic acid fragment may be simply referred to as a nucleic acid) having a specific nucleotide polymorphism site; and by detecting nuclease activity and ligase activity using nuclease activity and ligase activity.

More specifically, when only the uncomplementary portion of the nucleotide polymorphism site of the second oligonucleotide is accurately deleted by 5' exonuclease or 3' exonuclease, each oligonucleotide can be bound by ligase. The invention is accomplished by the above findings.

Namely, the present invention comprises the following.
1. A method for identifying a nucleotide polymorphism of a nucleotide-polymorphism-containing chromosome or nucleic acid fragment in a sample, which comprises the steps of:
   (1) preparing a first oligonucleotide that has a nucleotide polymorphism sequence site of the chromosome or nucleic acid fragment at the 3' end, and a second oligonucleotide that has a sequence position adjacent to the 3' site of the first oligonucleotide and hybridizes with the chromosome or nucleic acid fragment when the first oligonucleotide is hybridized with the chromosome or nucleic acid fragment, and that contains at least one base on the 5' end that does not hybridize with the chromosome or nucleic acid fragment;
   (2) hybridizing the first oligonucleotide and the second oligonucleotide with a specific nucleic acid;
   (3) binding each oligonucleotide by applying ligase activity, after using nuclease activity to delete the nucleotide sequence site that forms no base pairs, which is formed in the second oligonucleotide at the nucleotide polymorphism sequence when each olignucleotide is hybridized; and
   (4) detecting whether the first oligonucleotide and second oligonucleotide have formed a single chain.
2. A method for identifying the nucleotide polymorphism of a nucleotide-polymorphism-containing chromosome or nucleic acid fragment in a sample, which comprises the steps of:
   (1) preparing a first oligonucleotide that has a nucleotide polymorphism sequence site of the chromosome or nucleic acid fragment at the 5' end, and a second oligonucleotide, when the first oligonucleoitde is hybridized with the chromosome or nucleic acid fragment, that has a sequence position adjacent to the 5' site of the first oligonucleotide and hybridizes with the chromosome or nucleic acid fragment and that contains at least one base on the 3' end that does not hybridize with the chromosome or nucleic acid fragment;
   (2) hybridising the first oligonucleotide and the second oligonucleotide with a specific nucleic acid;
   (3) binding each oligonucleotide by applying ligase activity, after using nuclease activity to delete the nucleotide sequences that form no base pairs such as those that are formed in the second oligonucleotide at the nucleotide polymorphism sequence site when each oligonucleotide is hybridized; and
   (4) detecting whether the first oligonucleotide and second oligonucleotide have formed a single chain.
3. The method according to item 1 or 2, which detects nucleotide polymorphisms of a nucleotide-polymorphism-containing specific nucleic acid sequence in a sample, wherein an enzyme having nuclease activity and an enzyme having ligase activity are used sequentially and/or simultaneously.
4. The method according to item 1 or item 2, wherein the nucleotide polymorphism contains at least one member selected from the group consisting of a single nucleotide polymorphism, an inserted polymorphism, and a deleted polymorphism.
5. The method according to any one of items 1 to 4, wherein the enzyme having nuclease activity is at least one type of enzyme selected from the group consisting of Mung bean nuclease, S1 nuclease, and exonucleases I-VII.
6. The method according to any one of items 1 to 4, wherein the enzyme having ligase activity is at least one type of enzyme selected from the group consisting of T4DNA ligase, Ecoli DNA ligase, and RNA ligase.
7. The method according to any one of items 1 to 6, wherein the enzyme having nuclease activity and the enzyme having ligase activity are heat-resistant enzymes.
8. The method according to item 7, wherein the heat-resistant enzymes are derived from Tth, Taq, KOD, or Pfu.
9. The method according to any one of items 1 to 4, wherein the enzyme having nuclease activity is DNA polymerase.
10. The method according to item 9, wherein the DNA polymerase is a heat-resistant enzyme derived from Tth, Taq, KOD, or Pfu.
11. The method according to item 1 or 2, wherein a single oligonucleotide obtained by binding each oligonucleotide is detected using a detection probe.
12. The method according to item 1 or 2, wherein the nucleotide polymorphism sequence site has a single base and the base is anticipated to be a nucleotide polymorphism.
13. The method according to item 1 or 2, wherein bases that form no base pairs in the nucleotide polymorphism sequence site that is formed when each oligonucletoide is hybridized are deleted by nuclease activity, then each oligonucleotide is formed into a single oligonucleotide by applying ligase activity, and these processes are repeatedly conducted.
14. The method according to item 1 or 2, wherein at least one of the first oligonucleotide and the second oligonucleotide is labelled in advance.
15. The method according to item 14, wherein the labelling is conducted using at least one member selected from the group consisting of enzyme, biotin, fluorescent material, hapten, antigen, antibody, radioactive material, luminophore, and specific nucleic acid sequences.
16. The method according to item 11, which comprises a second oligonucleotide, wherein the sequence site that forms no base pairs in the nucleotide polymorphism sequence site of the second oligonucleotide and the first oligonucleotide hybridized with a target sequence are bound to different labels, and detection of whether or not the sequences are deleted can be conducted based on the two types of labels when the sequence that forms no base pairs is deleted by nuclease activity.
17. A kit for identifying the nucleotide polymorphism of a nucleotide-polymorphism-containing chromosome or nucleic acid fragment in a sample, wherein the kit comprises
   (i) at least one type of first oligonucleotide selected from a group consisting of a wild-type first oligonucleotide and one or two types of a first oligonucleotide that has a nucleotide polymorphism sequence site of the chromosome or nucleic acid fragment at the 3' end;
   (ii) a second oligonucleotide that is complementary to the chromosome or nucleic acid fragment of a strand chain with which the first oligonucleotide is hybridized and that has a sequence position adjacent to the 3' site of the first oligonucleotide and that contains at least one base on the 5' end that does not hybridize with the nucleotide polymorphism sequence site;
   (iii) at least one type of enzyme having nuclease activity or ligase activity; and
   (iv) a detection probe, wherein the detection probe can detect hybridized products of first oligonucleotides and second oligonucleotides obtained by applying ligase activity, after using nuclease activity to delete the nucleotide sequences that do not form base pairs in the nucleotide polymorphism sequence site such as those that are formed in the polymorphism sequence site when each oligonucleotide is hybridized.
18. A kit for identifying the nucleotide polymorphism of a nucleotide-polymorphism-containing chromosome or nucleic acid fragment in a sample, wherein the kit comprises
   (v) at least one type of first oligonucleotide selected from a group consisting of a wild-type first oligonucleotide and one or two types of a first oligonucleotide that has a nucleotide polymorphism sequence site of the chromosome or nucleic acid fragment at the 5' end;
   (vi) a second oligonucleotide that is complementary to the chromosome or nucleic acid fragment of a strand chain with which the first oligonucleotide is hybridized and that has a sequence position adjacent to the 5' site of the first oligonucleotide and that contains at least one base on the 3' end that does not hybridize with the nucleotide polymorphism sequence site;
   (vii) at least one type of enzyme having nuclease activity or ligase activity; and
   (viii) a detection probe, wherein the detection probe can detect hybridized products of first oligonucleotides and second oligonucleotides obtained by applying ligase activity, after using nuclease activity to delete the nucleotide sequences that do not form base pairs in the nucleotide polymorphism sequence site such as those that are formed in the polymorphism sequence site when each oligonucleotide is hybridized.

The present invention is described in more detail below. There are no particular limitations on the chromosome or fragment thereof having a specific polymorphism site that is contained in a sample, as long as it is a target nucleic acid containing a nucleotide polymorphism site that carries the objective genetic information. Examples of such target nucleic acids include Alu sequences, exons and introns that are genes encoding proteins, promoters, etc. More specific examples include genetic materials that affect various diseases, including hereditary diseases, drug metabolism, and lifestyle disorders (high blood pressure, diabetes, etc.). Examples include the ACE gene in the case of high blood pressure.

In the present invention, a polymorphism nucleic acid of a chromosome or nucleic acid fragment includes a wild-type nucleic acid having at least one nucleotide replaced by another nucleotide due to point mutation, and a nucleic acid containing an inserted or deleted sequence in part of the wild-type nucleic acid. It is known that such nucleotide polymorphisms affect the constitution and the like. The method of the present invention is designed to investigate whether or not nucleic acids in a sample have such anticipated polymorphisms.

The method for detecting nucleotide polymorphisms of the present invention is characterized in that nuclease activity and ligase activity are used sequentially and/or simultaneously. The activities can be applied by enzymes, and it is also possible to use an enzyme having both activities.

Specifically, examples of enzymes having nuclease activity include Mung bean nuclease, S1 nuclease, exonucleases I-VII, etc., and examples of enzymes having ligase activity include T4DNA ligase, E.coli DNA ligase, RNA ligase, etc.

It is preferable that the enzymes having nuclease activity and enzymes having ligase activity be heat-resistant enzymes. Preferably, the heat-resistant enzymes are derived from Tth, Taq, KOD, or Pfu.

It is also preferable that the enzyme having nuclease activity be DNA polymerase. More specifically, it is preferable that the DNA polymerase be a heat-resistant enzyme derived from Tth, Taq, KOD, or Pfu.

The specific mode of the method for detecting nucleotide polymorphisms of the present invention is such that the method identifying the nucleotide polymorphisms of a nucleotide-polymorphism-containing chromosome or nucleic acid fragment in a sample comprises the steps of:
(1) preparing a first oligonucleotide that has a nucleotide polymorphism sequence site of the chromosome or nucleic acid fragment at the 3' end, and a second oligonucleotide that has a sequence position adjacent to the 3' site of the first oligonucleotide and hybridizes with the chromosome or nucleic acid fragment when the first oligonucleotide is hybridized with the chromosome or nucleic acid fragment, and that contains at least one base on the 5' end that does not hybridize with the chromosome or nucleic acid fragment;
(2) hybridizing the first oligonucleotide and the second oligonucleotide with a specific nucleic acid;
(3) binding each oligonucleotide by applying ligase activity, after using nuclease activity to delete the nucleotide sequence site that forms no base pairs, which is formed in the second oligonucleotide at the nucleotide polymorphism sequence when each olignucleotide is hybridized; and
(4) detecting whether the first oligonucleotide and second oligonucleotide have formed a single chain.

In the alternative, the method for identifying the nucleotide polymorphism of a nucleotide-polymorphism-containing chromosome or nucleic acid fragment in a sample comprises the steps of:
(1) preparing a first oligonucleotide that has a nucleotide polymorphism sequence site of the chromosome or nucleic acid fragment at the 5' end, and a second oligonucleotide, when the first oligonucleoitde is hybridized with the chromosome or nucleic acid fragment, that has a sequence position adjacent to the 5' site of the first oligonucleotide and hybridizes with the chromosome or nucleic acid fragment and that contains at least one base on the 3' end that does not hybridize with the chromosome or nucleic acid fragment;
(2) hybridising the first oligonucleotide and the second oligonucleotide with a specific nucleic acid;
(3) binding each oligonucleotide by applying ligase activity, after using nuclease activity to delete the nucleotide sequences that form no base pairs such as those that are formed in the second oligonucleotide at the nucleotide polymorphism sequence site when each oligonucleotide is hybridized; and
(4) detecting whether the first oligonucleotide and second oligonucleotide have formed a single chain.

Specifically, the first oligonucleotide is an oligonucleotide having bases complementary to the site of the sequence where the nucleotide polymorphism is anticipated. Preferably, the site where the nucleotide polymorphism is anticipated is the 3' or 5' end of the first nucleotide.

When the first oligonucleotide is closer to the 5' end than the second oligonucleotide, the nucleotide polymorphism site is on the 3' end of the first oligonucleotide. When the first oligonucleotide is closer to the 3' end than the second oligonucleotide, the nucleotide polymorphism site is on the 5' end of the first nucleotide, and the first nucleotide further comprises a phosphoric acid group at 5'.

The second oligonucleotide is an oligonucleotide having bases not complementary to the sequence site where the nucleotide polymorphism is anticipated. The second oligonucleotide overlaps with the first oligonucleotide on at least one of the bases.

The length of each oligonucleotide in the present invention is 13-35 bases, and preferably 16-30 bases.

For example, when the 3' end base of the first oligonucleotide is designed to be an oligonucleotide where a polymorphism site is anticipated, in the case of a wild-type first oligonucleotide, a base complementary to the above-described base that is a wild-type nucleic acid is arranged, and, in the case of a polymorphism first oligonucleotide, a base complementary to the above-described base that is a polymorphism nucleic acid is arranged. Furthermore, when the 5' end of the second oligonucleotide is designed to overlap with the first oligonucleotide in one base, the overlapping base of the second oligonucleotide should be selected from the non-complementary bases both in the wild-type case and polymorphism case. By selecting a non-complementary base in both the wild type and polymorphism cases, it becomes sufficient to prepare only one kind of second oligonucleotide. The examples of possible combinations are as follows:

**Table 1**

| | | | | | | | | | | | | |
|---|---|---|---|---|---|---|---|---|---|---|---|---|
| Wild-type nucleic acid | A | | | G | | | C | | | T | | |
| 3' end of wild-type first oligonucleotide | T | | | C | | | G | | | A | | |
| Nucleic acid of polymorphism | G | C | T | A | T | C | A | T | G | G | C | A |
| 3' end of polymorphism first oligonucleotide | C | G | A | T | A | G | T | A | C | C | G | T |
| 5' end of second oligonucleotide | A | A | G | G | G | A | C | C | A | T | T | G |
| | G | C | C | A | T | T | A | T | T | G | C | C |

When such a design is employed according to the above-described principle, only in the combinations of wild-type first oligonucleotide/second oligonucleotide/wild-type nucleic acid and polymorphism first nucleotide/second oligonucleotide/polymorphism nucleic acid, the first oligonucleotide and the second oligonucleotide are bound into a single chain, since they completely correspond to each other except in the overlapped site of the second oligonucleotide. However, in the combinations of wild-type first oligonucleotide/second oligonucleotide/polymorphism nucleic acid and polymorphism first nucleotide/second oligonucleotide/wild-type nucleic acid, binding by ligase activity does not occur, since the 3' end of the first oligonucleotide is not complementary, the nuclease activity does not function, and the overlapped site of the second oligonucleotide is not deleted.

### Nuclease activity and ligase activity

In the present invention, the wild-type first oligonucleotide and the polymorphism first oligonucleotide are applied to a sample separately or simultaneously.
i) The aforementioned first oligonucleotide and second oligonucleotide are hybridized with a chromosome or nucleic acid fragment containing a nucleotide polymorphism. The hybridization conditions can be those generally employed. For example, hybridization can be performed following the steps described in Molecular Cloning.
ii) The overlapped site of the second oligonucleotide is cut using nuclease activity. Examples of usable enzymatic activities are as described above. Among those, exonuclease activity is preferable and exonucleases such as DNA polymerase or the like are preferably used.
   When the 5' end of the second oligonucleotide is overlapped with the first oligonucleotide, the overlapped site of the second oligonucleotide is cut using a 5' exonuclease to make a phosphoric acid group to be revealed.
   When the 3' end of the second oligonucleotide is overlapped with the first oligonucleotide, the overlapped site of the second oligonucleotide is cut using a 3' exonuclease.
iii) At the same time, an oligonucleotide at the 5' end and an oligonucleotide at the 3' end are bound into a single oligonucleotide using ligase activity.

In the present invention, if the site corresponding to the nucleotide polymorphism of the first oligonucleotide is not complementary, even when cut by an endnuclease, it is impossible to bind it into a single oligonucleotide using ligase activity, since the site corresponding to the nucleotide polymorphism is not complementary.

When nuclease activity and ligase activity are applied to a sample nucleic acid using a wild-type first oligonucleotide, a single chain is formed if the sample nucleic acid is of the wild type but no reaction occurs if the sample nucleic acid is a polymorphism. On the other hand, when nuclease activity and ligase activity are applied to a sample nucleic acid using a polymorphism first oligonucleotide, a single chain is formed if the sample nucleic acid is a polymorphism but no reaction occurs if the sample nucleic acid is of the wild type. Consequently, it is possible to accurately determine whether a sample nucleic acid is of the wild type or a polymorphism by splitting a single sample into two batches and investigating whether a reaction occurs using the wild type with one batch and the polymorphism with the other. In particular, humans and other higher organisms inherit two forms of each gene, one from the mother and one from the father, and this method makes it possible to determine whether a sample gene is wild-type homozygous, polymorphism homozygous, or heterozygous. In the case where the sample gene is heterozygous, both the wild-type gene and polymorphism gene are present, so that the reaction occurs both where the wild type is used and polymorphism is used.

By applying different labels to the wild-type first oligonucleotide and the polymorphism first oligonucleotide, it becomes unnecessary to split a single sample into two batches.

If the target nucleic acid is not in sufficient quantity for detection, the nucleic acid fragment containing the aforementioned polymorphism sequence can be amplified by the amplification reaction described below prior to the above-described hybridization.

There is no limitation on the amplification method and it is possible to employ a method such as PCR (Polymerase chain reaction: Japanese Examined Patent Publication No. 1992-67960, Japanese Examined Patent Publication No. 1992-67957), NASBA (Nucleic acid sequence-based amplification method: Nature Vol. 350 p. 91 (1991)), LCR (WO89/12696, Japanese Unexamined Patent Publication No. 1990-2934, etc.), SDA (Strand Displacement Amplification: Nucleic Acid Research Vol. 20 p. 1691 (1992)), RCR (WO90/01069), TMA (Transcription Mediated amplification Method: J. Clin. Microbiol. Vol. 31 p. 3270 (1993)), etc.

### Detection

The oligonucleotide of a single chain obtained by the above reaction is detected in the following manner using a probe.

It is preferable that the probes used in the present invention contain nucleotide polymorphisms, and detection signals vary depending on the kind of nucleotide polymorphism. Preferably, the probe should have a nucleotide sequence having at least 15 or more continuous bases and more preferably 18 or more continuous bases. Preferably, the probe has a sequence complementary to both a portion that contains the nucleotide polymorphism site of the first oligonucleotide and a portion of the second oligonucleotide. The probe can be DNA, RNA, or PNA, as long as it forms a chain complementary to a specific nucleic acid sequence, and it can be prepared by chemical synthesis or by a biological method. The probe can be synthesized by the phosphoamidite method using a 391 Perkin-Elmer DNA synthesizer. Purification can be conducted by FPLC using a MONO-Q column or a reversed column. Alternative methods include the phosphoric acid triester method, H-phosphonate method, thiophosphite method, etc.

It is preferable that the first oligonucleotide or the second oligonucleotide be labeled in advance. In this case, a modified base can be introduced by inserting a nucleotide that contains biotin, a linker arm, a fluorescent material, etc., oligo dGTP, oligo dATP, oligo dTTP, oligo dCTP, or the like to the 5' end or 3' end. Alternatively, a modified base can be introduced by substituting the nucleotide in the oligonucleotide sequence with a nucleotide containing biotin, a linker arm, a fluorescent material, or the like, and conducting synthesis. It is also possible to introduce known oligonucleotide labels, such as ³²P, ³⁵S, and like radioactive substances; ALP, POD, and like enzymes; FITC, HEX, 6-FAM, TET, and like fluorescent materials, etc., to the synthesized nucleotide.

Specifically, for example, the first oligonucleotide and the second oligonucleotide labeled with biotin or the like are hybridized with specific nucleic acids, dissociated, and hybridized with, for example, a polymorphism-specific probe bound to a solid phase, such as a micro titer plate. Thereafter, the nucleic acids not hybridized are washed out. Detection is then conducted to determine whether the nucleic acid is hybridized with the probe according to the label of the first oligonucleotide hybridized with the probe, and the type of polymorphism, such as wild type, variant type, or mixed type, is identified according to the ratio of the detection signal of each probe.

By applying different labels, such as fluorescent labels having different colors, to wild-type and polymorphism oligonucleotides, detection can be performed in wells on a single plate.

### Kit

In the present invention, a kit is characterized as containing
(i) at least one type of first oligonucleotide selected from a group consisting of a wild-type first oligonucleotide and one or two types of a first oligonucleotide that has a nucleotide polymorphism sequence site of the chromosome or nucleic acid fragment at the 3' end;
(ii) a second oligonucleotide that is complementary to the chromosome or nucleic acid fragment of a strand chain with which the first oligonucleotide is hybridized and that has a sequence position adjacent to the 3' site of the first oligonucleotide and that contains at least one base on the 5' end that does not hybridize with the nucleotide polymorphism sequence site
(iii) at least one type of enzyme having nuclease activity or ligase activity; and
(iv) a detection probe, wherein the detection probe can detect hybridized products of first oligonucleotides and second oligonucleotides obtained by applying ligase activity, after using nuclease activity to delete the nucleotide sequences that do not form base pairs in the nucleotide polymorphism sequence site such as those that are formed in the polymorphism sequence site when each oligonucleotide is hybridized.
In another aspect, a kit is characterized as containing
(v) at least one type of first oligonucleotide selected from a group consisting of a wild-type first oligonucleotide and one or two types of a first oligonucleotide that has a nucleotide polymorphism sequence site of the chromosome or nucleic acid fragment at the 5' end;
(vi) a second oligonucleotide that is complementary to the chromosome or nucleic acid fragment of a strand chain with which the first oligonucleotide is hybridized and that has a sequence position adjacent to the 5' site of the first oligonucleotide and that contains at least one base on the 3' end that does not hybridize with the nucleotide polymorphism sequence site;
(vii) at least one type of enzyme having nuclease activity or ligase activity; and
   a detection probe, wherein the detection probe can detect hybridized products of first oligonucleotides and second oligonucleotides obtained by applying ligase activity, after using nuclease activity to delete the nucleotide sequences that do not form base pairs in the nucleotide polymorphism sequence site such as those that are formed in the polymorphism sequence site when each oligonucleotide is hybridized.

The first oligonucleotide and the second oligonucleotide may be labeled in advance by the aforementioned enzyme, biotin, fluorescent material, hapten, antigen, antibody, radioactive material, or luminophore.

### BEST MODE FOR CARRYING OUT THE INVENTION

The present invention is described in more detail below based on examples. However, the present invention is not limited by the following examples.

### Example 1: Detection of Nucleotide Polymorphism in the ACE gene

### (1) Synthesis of primers for amplifying the ACE gene fragment and oligonucleotide for detecting polymorphism

Oligonucleotides (primers 1, 2) having the nucleotide sequences shown by Seq. Nos. 1 and 2 that have the same sequence as that of the human ACE gene, and oligonucleotides (detection probes 3, 4, 5) having the nucleotide sequences shown by Seq. Nos. 3, 4, and 5 were synthesized by the phosphoamidite method using a 392 Perkin-Elmer DNA synthesizer. The detection probes 3 and 4 have sequences complementary to that of the human ACE gene. However, each of them has a wild-type or polymorphism base corresponding to the nucleotide polymorphism site at the 3' end, and biotin is connected to the 5' side thereof. The detection probe 5 has a sequence complementary to that of the human ACE gene adjacent to the 3' end of the detection probes 3 and 4; however, the single base at its 5' end corresponds to the nucleotide polymorphism site and is not complementary to the wild type or the polymorphism. Synthesis was performed according to the manual, and the various oligonucleotides were deprotected overnight at 55 °C with ammonia water. Purification of the oligonucleotides was conducted using a Perkin-Elmer OPC column.

### (2) Synthesis of oligonucleotide having a linker arm

An oligonucleotide having the nucleotide sequence shown by Seq. No. 6 (A probe) and an oligonucleotide having the nucleotide sequence shown by Seq. No. 7 (T probe) were synthesized by the phosphoamidite method using a 392 Perkin-Elmer DNA synthesizer. Both the A probe and T probe are complementary to the sequence of the human ACE gene and specific in the nucleotide polymorphism sites thereof.

During the synthesizing process, according to the synthesizing method disclosed in Japanese Unexamined Patent Publication No. 1985-500717, uridine having a linker arm at its 5-position prepared by chemically synthesizing deoxyuridine was introduced into the aforementioned oligonucleotide. The synthesized linker oligonucleotides were deprotected overnight with ammonia water at 50 °C and purified using a Perkin-Elmer OPC column.

### (3) Binding of probe oligonucleotide to microtiter plate

The probe oligonucleotide synthesized in item (2) described above was bound to the inner surface of a microtiter plate through the linker arm thereof. The oligonucleotide was diluted with a solution of 50 mM of boric acid buffer solution (pH 10) and 100 mM of MgCl₂ in a manner such that the concentration became 0.05 pmol/µl, and 100 µl of the resultant solution was dispersed into each well of the microtiter plate (MicroFLUOR B, Dynatech Corp) and allowed to stand for about 15 hours at room temperature, binding the linker oligonucleotide to the inner surfaces of the microtiter plate. These were then substituted with 0.1 pmol of dNTP, 0.5% of PVP (polyvinyl pyrolidone), and 5 X SSC, then blocked for about 2 hours at room temperature to suppress non-specific reactions, and finally washed in 1 x SSC and dried.

### (4) Amplification of the human ACE gene fragment by PCR

Using DNA solutions extracted from human keukocytes as samples, human ALDH2 gene fragments were amplified by adding the following reagents under the conditions described below.
Reagents: A 25-µl solution containing the following reagents was prepared.

| | |
|---|---|
| Primer 1 | 10 pmol |
| Primer 2 | 10 pmol |
| X 10 buffer | 2.5 µl |
| 2 mM dNTP | 2.5 µl |
| Tth DNA polymerase | 1 U |
| Extracted DNA solution | 100 ng |

### Amplification Conditions

94 °C for 2 minutes
94 °C for 1 minute, 57 °C for 2 minutes, 75 °C for 1.5 minutes (35 cycles)

### (5) Detecting reaction using detection oligonucleotide

Alkaline phosphatase U derived from shrimp was added to 5 µl of the amplification reaction solution described in item (4) and kept at 37 °C for 15 minutes and at 80 °C for 15 minutes. Then, a 20-µl solution containing the following reagents was prepared.

| | |
|---|---|
| Detection oligo 3 or 4 | 10 pmol |
| Detection oligo 5 | 10 pmol |
| X 10 buffer | 2 µl |
| Tth DNA polymerase | 1 U |
| Tth DNA ligase | 1 U |
| Amplification reaction solution | 5 µl |

### Detection conditions

94 °C for 2 minutes
94 °C for 1 minute, 65°C for 2 minutes (5 cycles)

### (6) Hybridization in microtiter plates

10 µl of 0.6 N NaOH was added to 10 µl of the detecting reaction solution described in item (5) for denaturing the DNA. Then, the resultant solution was added to 80 µl of solution comprising 200 mM of citric acid-phosphoric acid buffer solution (pH 6.0), 2 % SDS (sodium dodecyl sulfate), 750 mM of NaCl, and 0.1 % NaN₃ and poured into the micotiter plate described in item (3) to which capture probes were bound. These were covered with liquid paraffin to prevent evaporation, and shaken for 30 minutes at 50 °C. In this way, a nucleic acid fragment in which the nuclear oligonucleotide has been hybridized by a detecting reaction, partly deleted by nuclease, and bound by ligase was specifically captured on the microtiter plate by the fixed probes.

Then, these were substituted with 2 X SSC (pH 7.0), 1% SDS and also covered with liquid paraffin to prevent evaporation, followed by shaking for 20 minutes at 55 °C. Next, these were substituted with 100 µl of a solution obtained by diluting streptoavidin (DAKO: D0396) labeled with alkaline phosphatase by 2,000 times with a solution of 50 mM of tris-hydrochloric acid buffer (pH 7.5) and 1 % BSA solution, and shaken for 15 minutes at 37 °C. This served to bind the alkaline phosphatase-labeled streptoavidin specifically to the biotin of the captured DNA. After washing this three times in 250 µl of a solution containing 50 mM of tris-hydrochloric acid buffer (pH 7.5) and 0.025 % Tween 20 solution, 50 µl of dioxetan compound (Trade name Lumiphos 480, Lumigen Co.), which is the luminescent base of alkaline phosphatase, was added thereto. After keeping it at 37 °C for 15 minutes, luminescence was measured in a dark room using a photon counter (Hamamatsu Photonics KK).

All of these steps were performed automatically with an automated DNA probe measurement system (see the Nihon Rinshokensa Jidokagakukai Kaishi (Journal of the Japanese Association for Automation of Clinical Testing) Vol. 20, Page 728 (1995)), and took about 2.5 hours.

### (7) Results of the measurement and examination of the human ACE gene polymorphism

Table 1 shows the detection results of human ACE gene polymorphisms that were reacted in the above-described Item (5) and detected in Item (6). The values indicate luminousity (cps: count/second). The A signal represents the detection signal of an amplified nucleic acid fragment that was reacted with an A probe. The T signal represents the detection signal of an amplified nucleic acid fragment that was reacted with a T probe.

**Table 2**

| | Sample type | T signal | A signal |
|---|---|---|---|
| Detection oligos 3, 5 | T/T | 22650 | 170 |
| | T/A | 17250 | 12620 |
| | A/A | 260 | 80 |
| Detection oligos 4, 5 | T/T | 110 | 450 |
| | T/A | 17880 | 11690 |
| | A/A | 120 | 13230 |

As shown in Table 1, the single nucleotide polymorphism of the ACE gene can be identified by the signals obtained by each probe.

The present invention provides a method for accurately and readily detecting a polymorphism in a sample nucleic acid. The method of the present invention is free from false positives and enables readable identification between homozygosis and heterozygosis, which was difficult using the known methods.

## Claims

1. A method for identifying a nucleotide polymorphism of a nucleotide-polymorphism-containing chromosome or nucleic acid fragment in a sample, which comprises the steps of:
(1) preparing a first oligonucleotide that has a nucleotide polymorphism sequence site of the chromosome or nucleic acid fragment at the 3' end, and a second oligonucleotide that has a sequence position adjacent to the 3' site of the first oligonucleotide and hybridizes with the chromosome or nucleic acid fragment when the first oligonucleotide is hybridized with the chromosome or nucleic acid fragment, and that contains at least one base on the 5' end that does not hybridize with the chromosome or nucleic acid fragment;
(2) hybridizing the first oligonucleotide and the second oligonucleotide with a specific nucleic acid;
(3) binding each oligonucleotide by applying ligase activity, after using nuclease activity to delete the nucleotide sequence site that forms no base pairs, which is formed in the second oligonucleotide at the nucleotide polymorphism sequence when each olignucleotide is hybridized; and
(4) detecting whether the first oligonucleotide and second oligonucleotide have formed a single chain.

2. A method for identifying the nucleotide polymorphism of a nucleotide-polymorphism-containing chromosome or nucleic acid fragment in a sample, which comprises the steps of:
(1) preparing a first oligonucleotide that has a nucleotide polymorphism sequence site of the chromosome or nucleic acid fragment at the 5' end, and a second oligonucleotide, when the first oligonucleoitde is hybridized with the chromosome or nucleic acid fragment, that has a sequence position adjacent to the 5' site of the first oligonucleotide and hybridizes with the chromosome or nucleic acid fragment and that contains at least one base on the 3' end that does not hybridize with the chromosome or nucleic acid fragment;
(2) hybridising the first oligonucleotide and the second oligonucleotide with a specific nucleic acid;
(3) binding each oligonucleotide by applying ligase activity, after using nuclease activity to delete the nucleotide sequences that form no base pairs such as those that are formed in the second oligonucleotide at the nucleotide polymorphism sequence site when each oligonucleotide is hybridized; and
(4) detecting whether the first oligonucleotide and second oligonucleotide have formed a single chain.

3. The method according to claim 1 or 2, which detects nucleotide polymorphisms of a nucleotide-polymorphism-containing specific nucleic acid sequence in a sample, wherein an enzyme having nuclease activity and an enzyme having ligase activity are used sequentially and/or simultaneously.

4. The method according to claim 1 or claim 2, wherein the nucleotide polymorphism contains at least one member selected from the group consisting of a single nucleotide polymorphism, an inserted polymorphism, and a deleted polymorphism.

5. The method according to any one of claims 1 to 4, wherein the enzyme having nuclease activity is at least one type of enzyme selected from the group consisting of Mung bean nuclease, S 1 nuclease, and exonucleases I-VII.

6. The method according to any one of claims 1 to 4, wherein the enzyme having ligase activity is at least one type of enzyme selected from the group consisting of T4DNA ligase, Ecoli DNA ligase, and RNA ligase.

7. The method according to any one of claims 1 to 6, wherein the enzyme having nuclease activity and the enzyme having ligase activity are heat-resistant enzymes.

8. The method according to claim 7, wherein the heat-resistant enzymes are derived from Tth, Taq, KOD, or Pfu.

9. The method according to any one of claims 1 to 4, wherein the enzyme having nuclease activity is DNA polymerase.

10. The method according to claim 9, wherein the DNA polymerase is a heat-resistant enzyme derived from Tth, Taq, KOD, or Pfu.

11. The method according to claim 1 or 2, wherein a single oligonucleotide obtained by binding each oligonucleotide is detected using a detection probe.

12. The method according to claim 1 or 2, wherein the nucleotide polymorphism sequence site has a single base and the base is anticipated to be a nucleotide polymorphism.

13. The method according to claim 1 or 2, wherein bases that form no base pairs in the nucleotide polymorphism sequence site that is formed when each oligonucletoide is hybridized are deleted by nuclease activity, then each oligonucleotide is formed into a single oligonucleotide by applying ligase activity, and these processes are repeatedly conducted.

14. The method according to claim 1 or 2, wherein at least one of the first oligonucleotide and the second oligonucleotide is labelled in advance.

15. The method according to claim 14, wherein the labelling is conducted using at least one member selected from the group consisting of enzyme, biotin, fluorescent material, hapten, antigen, antibody, radioactive material, luminophore, and specific nucleic acid sequences.

16. The method according to claim 11, which comprises a second oligonucleotide, wherein the sequence site that forms no base pairs in the nucleotide polymorphism sequence site of the second oligonucleotide and the first oligonucleotide hybridized with a target sequence are bound to different labels, and detection of whether or not the sequences are deleted can be conducted based on the two types of labels when the sequence that forms no base pairs is deleted by nuclease activity.

17. A kit for identifying the nucleotide polymorphism of a nucleotide-polymorphism-containing chromosome or nucleic acid fragment in a sample, wherein the kit comprises
(i) at least one type of first oligonucleotide selected from a group consisting of a wild-type first oligonucleotide and one or two types of a first oligonucleotide that has a nucleotide polymorphism sequence site of the chromosome or nucleic acid fragment at the 3' end;
(ii) a second oligonucleotide that is complementary to the chromosome or nucleic acid fragment of a strand chain with which the first oligonucleotide is hybridized and that has a sequence position adjacent to the 3' site of the first oligonucleotide and that contains at least one base on the 5' end that does not hybridize with the nucleotide polymorphism sequence site;
(iii) at least one type of enzyme having nuclease activity or ligase activity; and
(iv) a detection probe, wherein the detection probe can detect hybridized products of first oligonucleotides and second oligonucleotides obtained by applying ligase activity, after using nuclease activity to delete the nucleotide sequences that do not form base pairs in the nucleotide polymorphism sequence site such as those that are formed in the polymorphism sequence site when each oligonucleotide is hybridized.

18. A kit for identifying the nucleotide polymorphism of a nucleotide-polymorphism-containing chromosome or nucleic acid fragment in a sample, wherein the kit comprises
(v) at least one type of first oligonucleotide selected from a group consisting of a wild-type first oligonucleotide and one or two types of a first oligonucleotide that has a nucleotide polymorphism sequence site of the chromosome or nucleic acid fragment at the 5' end;
(vi) a second oligonucleotide that is complementary to the chromosome or nucleic acid fragment of a strand chain with which the first oligonucleotide is hybridized and that has a sequence position adjacent to the 5' site of the first oligonucleotide and that contains at least one base on the 3' end that does not hybridize with the nucleotide polymorphism sequence site;
(vii) at least one type of enzyme having nuclease activity or ligase activity; and
(viii) a detection probe, wherein the detection probe can detect hybridized products of first oligonucleotides and second oligonucleotides obtained by applying ligase activity, after using nuclease activity to delete the nucleotide sequences that do not form base pairs in the nucleotide polymorphism sequence site such as those that are formed in the polymorphism sequence site when each oligonucleotide is hybridized.

## Patentansprüche

1. Verfahren zum Identifizieren eines Nucleotid-Polymorphismus eines Nucleotid-Polymorphismus-enthaltenden Chromosoms oder Nucleinsäurefragments in einer Probe, das die Schritte umfasst:
(1) Herstellen eines ersten Oligonucleotids, das eine Nucleotid-Polymorphismus-Sequenzstelle des Chromosoms oder Nucleinsäurefragments am 3'-Ende hat, und eines zweiten Oligonucleotids, das eine Sequenzposition angrenzend an die 3'-Stelle des ersten Oligonucleotids hat und das mit dem Chromosom oder Nucleinsäurefragment hybridisiert, wenn das erste Oligonucleotid mit dem Chromosom oder dem Nucleinsäurefragment hybridisiert wird, und das mindestens eine Base am 5'-Ende enthält, die nicht mit dem Chromosom oder Nucleinsäurefragment hybridisiert;
(2) Hybridisieren des ersten Oligonucleotids und des zweiten Oligonucleotids mit einer spezifischen Nucleinsäure;
(3) Binden jedes Oligonucleotids durch das Anwenden von Ligase-Aktivität nach Verwenden von Nuclease-Aktivität, um die Nucleotidsequenzstelle, die keine Basenpaare bildet, zu entfernen, die in dem zweiten Oligonucleotid an der Nucleotid-Polymorphismus-Sequenz gebildet wird, wenn jedes Oligonucleotid hybridisiert wird; und
(4) Nachweisen, ob das erste Oligonucleotid und das zweite Oligonucleotid eine einzelne Kette gebildet haben.

2. Verfahren zum Identifizieren des Nucleotid-Polymorphismus eines Nucleotid-Polymorphismus-enthaltenden Chromosoms oder Nucleinsäurefragments in einer Probe, das die Schritte umfasst:
(1) Herstellen eines ersten Oligonucleotids, das eine Nucleotid-Polymorphismus-Sequenzstelle des Chromosoms oder Nucleinsäurefragments am 5'-Ende hat, und eines zweiten Oligonucleotids, wenn das erste Oligonucleotid mit dem Chromosom oder Nucleinsäurefragment hybridisiert wird, das eine Sequenzstelle angrenzend an die 5'-Stelle des ersten Oligonucleotids hat und das mit dem Chromosom oder Nucleinsäurefragment hybridisiert, und das mindestens eine Base am 3'-Ende enthält, die nicht mit dem Chromosom oder Nucleinsäurefragment hybridisiert;
(2) Hybridisieren des ersten Oligonucleotids und des zweiten Oligonucleotids mit einer spezifischen Nucleinsäure;
(3) Binden jedes Oligonucleotids durch das Anwenden von Ligase-Aktivität nach Verwenden von Nuclease-Aktivität, um die Nucleotidsequenzen zu entfernen, die keine Basenpaare bilden, wie solche, die in dem zweiten Oligonucleotid an der Nucleotid-Polymorphismus-Sequenzstelle gebildet werden, wenn jedes Oligonucleotid hybridisiert wird; und
(4) Nachweisen, ob das erste Oligonucleotid und das zweite Oligonucleotid eine einzelne Kette gebildet haben.

3. Verfahren gemäß Anspruch 1 oder 2, das Nucleotid-Polymorphismen einer Nucleotid-Polymorphismus-enthaltenden spezifischen Nucleinsäuresequenz in einer Probe nachweist, wobei ein Enzym mit Nuclease-Aktivität und ein Enzym mit Ligase-Aktivität, aufeinanderfolgend und/oder gleichzeitig verwendet werden.

4. Verfahren gemäß Anspruch 1 oder Anspruch 2, wobei der Nucleotid-Polymorphismus mindestens ein Mitglied ausgewählt aus der Gruppe bestehend aus einem einzelnen Nucleotid-Polymorphismus, einen eingefügten Polymorphismus und einem entfernten Polymorphismus enthält.

5. Verfahren gemäß einem der Ansprüche 1 bis 4, wobei das Enzym mit Nuclease-Aktivität mindestens eine Enzymart ausgewählt aus der Gruppe bestehend aus Mung-Bohnen-Nuclease, S1-Nuclease, und Exonucleasen I-VII ist.

6. Verfahren gemäß einem der Ansprüche 1 bis 4, wobei das Enzym mit Ligase-Aktivität mindestens eine Enzymart ausgewählt aus der Gruppe bestehend aus T4DNA-Ligase, Ecoli-DNA-Ligase und RNA-Ligase ist.

7. Verfahren gemäß einem der Ansprüche 1 bis 6, wobei das Enzym mit Nuclease-Aktivität und das Enzym mit Ligase-Aktivität hitzebeständige Enzyme sind.

8. Verfahren gemäß Anspruch 7, wobei die hitzebeständigen Enzyme von Tth, Taq, KOD oder Pfu abgeleitet sind.

9. Verfahren gemäß einem der Ansprüche 1 bis 4, wobei das Enzym mit Nuclease-Aktivität DNA-Polymerase ist.

10. Verfahren gemäß Anspruch 9, wobei die DNA-Polymerase ein hitzebeständiges Enzym abgeleitet von Tth, Taq, KOD oder Pfu ist.

11. Verfahren gemäß Anspruch 1 oder 2, wobei ein einzelnes Oligonucleotid, das durch das Binden jedes Oligonucleotides erhalten wird, unter Verwendung einer Nachweissonde nachgewiesen wird.

12. Verfahren gemäß Anspruch 1 oder 2, wobei die Nucleotid-Polymorphismus-Sequenzstelle eine einzelne Base hat und es erwartet wird, dass die Base ein Nucleotid-Polymorphismus ist.

13. Verfahren gemäß Anspruch 1 oder 2, wobei Basen, die in der Nucleotid-Polymorphismus-Sequenzstelle, die geformt wird, wenn jedes Oligonucleotid hybridisiert wird, keine Basenpaare bilden, durch Nuclease-Aktivität entfernt werden, dann jedes Oligonucleotid durch Anwenden von Ligase-Aktivität in ein einzelnes Oligonucleotid umgeformt wird und diese Vorgänge wiederholt durchgeführt werden.

14. Verfahren gemäß Anspruch 1 oder 2, wobei mindestens eines von dem ersten Oligonucleotid und dem zweiten Oligonucleotid im Voraus markiert wird.

15. Verfahren gemäß Anspruch 14, wobei das Markieren durchgeführt wird unter Verwendung von mindestens einem Mitglied ausgewählt aus der Gruppe bestehend aus einem Enzym, Biotin, fluoreszierendem Material, Hapten, Antigen, Antikörper, radioaktivem Material, Luminophor und spezifischen Nucleinsäuresequenzen.

16. Verfahren gemäß Anspruch 11, das ein zweites Oligonucleotid umfasst, wobei die Sequenzstelle, die in der Nucleotid-Polymorphismus-Sequenzstelle des zweiten Oligonucleotids keine Basenpaare bildet, und das erste Oligonucleotid, das mit einer Zielsequenz hybridisiert, an unterschiedliche Marker gebunden sind, und der Nachweis, ob die Sequenzen entfernt sind oder ob nicht, durchgeführt werden kann basierend auf den zwei Arten von Markern, wenn die Sequenz, die keine Basenpaare bildet, durch Nuclease-Aktivität entfernt wird.

17. Kit zum Identifizieren des Nucleotid-Polymorphismus eines Nucleotid-Polymorphimus-enthaltenden Chromosoms oder Nucleinsäurefragments in einer Probe, wobei das Kit umfasst:
(i) mindestens eine Art eines ersten Oligonucleotids ausgewählt aus einer Gruppe bestehend aus einem Wildtyp eines ersten Oligonucleotids und einer oder zwei Arten eines ersten Oligonucleotids, das eine Nucleotid-Polymorphismus-Sequenzstelle des Chromosoms oder Nucleinsäurefragments am 3'-Ende hat;
(ii) ein zweites Oligonucleotid, dass komplementär zu dem Chromosom oder Nucleinsäurefragment eines Kettenstrangs ist, mit dem das erste Oligonucleotid hybridisiert ist, und das eine Sequenzstelle angrenzend an die 3'-Stelle des ersten Oligonucleotids hat und das mindestens eine Base am 5'-Ende enthält, die nicht mit der Nucleotid-Polymorphismus-Sequenzstelle hybridisiert;
(iii) mindestens eine Enzymeart mit Nuclease-Aktivität oder Ligase-Aktivität; und
(iv) eine Nachweissonde, wobei die Nachweissonde hybridisierte Produkte von ersten Oligonucleotiden und zweiten Oligonucleotiden nachweisen kann, die durch das Anwenden von Ligase-Aktivität erhalten wurden, nach Verwenden von Ligase-Aktivität, um die Nucleotidsequenzen zu entfernen, die in der Nucleotid-Polymorphismus-Sequenzstelle keine Basenpaare bilden, wie solche, die in der Polymorphismus-Sequenzstelle gebildet werden, wenn jedes Oligonucleotid hybridisiert wird.

18. Kit zum Identifizieren des Nucleotid-Polymorphismus eines Nucleotid-Polymorphimus-enthaltenden Chromosoms oder Nucleinsäurefragments in einer Probe, wobei das Kit umfasst:
(v) mindestens eine Art eines ersten Oligonucleotids ausgewählt aus der Gruppe bestehend aus einem Wildtyp eines ersten Oligonucleotids und einer oder zwei Arten eines ersten Oligonucleotids, das eine Nucleotid-Polymorphismus-Sequenzstelle des Chromosoms oder Nucleinsäurefragments am 5'-Ende hat;
(vi) ein zweites Oligonucleotid, das zu dem Chromosom oder Nucleinsäurefragment eines Kettenstrangs komplementär ist, mit dem das erste Oligonucleotid hybridisiert wird, und das eine Sequenzstelle angrenzend an die 5'-Stelle des ersten Oligonucleotids hat und das mindestens eine Base an dem 3'-Ende enthält, die nicht mit der Nucleotid-Polymorphismus-Sequenzstelle hybridisiert;
(vii) mindestens eine Enzymart mit Nuclease-Aktivität oder Ligase-Aktivität; und
(viii) eine Nachweissonde, wobei die Nachweissonde hybridisierte Produkte von ersten Oligonucleotiden und zweiten Oligonucleotiden nachweisen kann, die durch das Anwenden von Ligase-Aktivität erhalten wurden, nach Verwenden von Nuclease-Aktivität, um die Nucleotidsequenzen zu entfernen, die in der Nucleotid-Polymorphismus-Sequenzstelle keine Basenpaare bilden, wie solche, die in der Polymorphismus-Sequenzstelle gebildet werden, wenn jedes Oligonucleotid hybridisiert wird.

## Revendications

1. Procédé d'identification d'un polymorphisme nucléotidique sur un chromosome ou un fragment d'acide nucléique contenant un (des) polymorphisme(s) nucléotidique(s) dans un échantillon, qui comprend les étapes consistant à :
(1) préparer un premier oligonucléotide qui a à l'extrémité 3', un site polymorphe de la séquence nucléotidique du chromosome ou du fragment d'acide nucléique et un second oligonucléotide dont la séquence est en position adjacente au site en 3' du premier oligonucléotide et s'hybride avec le chromosome ou fragment d'acide nucléique lorsque le premier oligonucléotide est hybridé avec le chromosome ou fragment d'acide nucléique, et qui contient au moins une base à l'extrémité 5' qui ne s'hybride pas avec le chromosome ou fragment d'acide nucléique ;
(2) hybrider le premier oligonucléotide et le second oligonucléotide avec un acide nucléique spécifique ;
(3) lier chaque oligonucléotide en appliquant une activité ligase, après l'utilisation d'une activité nucléase pour déléter le site de séquence nucléotidique qui ne forme pas de paires de bases, qui est formé dans le second oligonucléotide au niveau de la séquence nucléotidique polymorphe lorsque chaque oligonucléotide est hybridé ; et
(4) détecter si le premier oligonucléotide et le second oligonucléotide ont formé une chaîne unique.

2. Procédé d'identification du polymorphisme nucléotidique d'un chromosome ou d'un fragment d'acide nucléique contenant un (des) polymorphisme(s) nucléotidiques dans un échantillon, qui comprend les étapes consistant à :
(1) préparer un premier oligonucléotide qui a un site polymorphe de la séquence nucléotidique du chromosome ou fragment d'acide nucléique à l'extrémité 5', et un second oligonucléotide, qui a, lorsque le premier oligonucléotide est hybridé avec le chromosome ou fragment d'acide nucléique, une séquence en position adjacente au site en 5' du premier oligonucléotide et s'hybride avec le chromosome ou fragment d'acide nucléique, et qui contient au moins une base sur l'extrémité 3' qui ne s'hybride pas avec le chromosome ou fragment d'acide nucléique ;
(2) hybrider le premier oligonucléotide et le second oligonucléotide avec un acide nucléique spécifique ;
(3) lier chaque oligonucléotide en appliquant une activité ligase, après avoir utiliser une activité nucléase pour déléter les séquences nucléotidiques qui ne forment pas de paires de bases telles que celles qui sont formées dans le second oligonucléotide au niveau du site polymorphe de la séquence nucléotidique lorsque chaque oligonucléotide est hybridé ; et
(4) détecter si le premier oligonucléotide et le second oligonucléotide ont formé une chaîne unique.

3. Procédé selon la revendication 1 ou 2, qui détecte des polymorphismes nucléotidiques d'une séquence d'acide nucléique spécifique contenant un (des) polymorphisme(s) nucléotidique(s) dans un échan-tillon, dans lequel une enzyme possédant une activité nucléase et une enzyme possédant une activité ligase sont utilisées séquentiellement et/ou simultanément.

4. Procédé selon la revendication 1 ou 2, dans lequel le polymorphisme nucléotidique contient au moins un membre choisi dans le groupe constitué par un polymorphisme d'un nucléotide unique, un polymorphisme inséré et un polymorphisme délété.

5. Procédé selon l'une quelconque des revendications 1 à 4, dans lequel l'enzyme possédant une activité nucléase est au moins un type d'enzyme choisi dans le groupe constitué par la nucléase du haricot Mungo, la nucléase S1 et les exo-nucléases I à VII.

6. Procédé selon l'une quelconque des revendications 1 à 4, dans lequel l'enzyme possédant une activité ligase est au moins un type d'enzyme choisi dans le groupe constitué de la T4 ADN ligase, l'ADN ligase de E. coli et une ARN ligase.

7. Procédé selon l'une quelconque des revendications 1 à 6, dans lequel l'enzyme possédant une activité nucléase et l'enzyme possédant une activité ligase sont des enzymes thermorésistantes.

8. Procédé selon la revendication 7, dans lequel les enzymes thermorésistantes sont dérivées de Tth, Taq, KOD ou Pfu.

9. Procédé selon l'une quelconque des revendications 1 à 4, dans lequel l'enzyme possédant une activité nucléase est une ADN polymérase.

10. Procédé selon la revendication 9, dans lequel l'ADN polymérase est une enzyme thermorésistante dérivée de Tth, Taq, KOD ou Pfu.

11. Procédé selon la revendication 1 ou 2, dans lequel un oligonucléotide unique obtenu par liaison de chaque oligonucléotide est détecté en utilisant une sonde de détection.

12. Procédé selon la revendication 1 ou 2, dans lequel le site polymorphe de la séquence nucléotidique a une seule base et la base est prévue d'être un polymorphisme nucléotidique.

13. Procédé selon la revendication 1 ou 2, dans lequel des bases qui ne forment pas des paires de bases au site polymorphe de la séquence nucléotidique qui est formé lorsque chaque oligonucléotide est hybridé, sont délétées par une activité nucléase, puis chaque oligo-nucléotide est transformé en un oligo-nucléotide unique en appliquant une activité ligase, et ces procédés sont conduits de manière répétée.

14. Procédé selon la revendication 1 ou 2, dans lequel au moins l'un du premier oligonucléotide et du second oligonucléotide est marqué à l'avance.

15. Procédé selon la revendication 14, dans lequel le marquage est conduit en utilisant au moins un membre choisi dans le groupe constitué d'enzyme, de biotine, de matériau fluorescent, d'haptène, d'antigène, d'anticorps, de matériau radioactif, de luminophore et de séquences d'acide nucléique spécifiques.

16. Procédé selon la revendication 11, qui comprend un second oligonucléotide, dans lequel le site de la séquence qui ne forme pas de paires de bases au niveau du site polymorphe de la séquence nucléotidique du second oligonucléotide et le premier oligonucléotide hybridé avec une séquence cible sont liés à des marqueurs différents, et la détection permettant de savoir si les séquences sont délétées ou non, peut être conduite en se basant sur les deux types de marqueurs lorsque la séquence qui ne forme pas de paires de bases est délétée par une activité nucléase.

17. Kit pour identifier le polymorphisme nucléotidique d'un chromosome ou fragment d'acide nucléique contenant un (des) polymorphisme(s) nucléo-tidiques dans un échantillon, où le kit comprend
(i) au moins un type de premier oligo-nucléotide choisi dans un groupe constitué par un premier oligonucléotide de type sauvage et d'un ou deux types d'un premier oligonucléotide qui a un site polymorphe de la séquence nucléotidique du chromosome ou fragment d'acide nucléique à l'extrémité 3' ;
(ii) un second oligonucléotide qui est complémentaire d'une chaîne de brin du chromosome ou fragment d'acide nucléique avec laquelle le premier oligonucléotide est hybridé et qui a une séquence en position adjacente au site en 3' du premier oligo-nucléotide et qui contient au moins une base sur l'extrémité 5' qui ne s'hybride pas avec le site polymorphe de la séquence nucléotidique ;
(iii) au moins un type d'enzyme possédant une activité nucléase ou un activité ligase ; et
(iv) une sonde de détection, laquelle peut détecter des produits issus de l'hybridation des premiers oligonucléotides et des seconds oligonucléotides obtenus en appliquant une activité ligase après avoir utilisé une activité nucléase pour déléter les séquences nucléotidiques qui ne forment pas des paires de bases au site polymorphe de la séquence nucléotidique, telles que celles qui sont formées au niveau du site polymorphe de la séquence lorsque chaque oligonucléotide est hybridé.

18. Kit pour identifier le polymorphisme nucléotidique d'un chromosome ou fragment d'acide nucléique contenant un (des) polymorphisme(s) nucléo-tidique(s) dans un échantillon, lequel kit comprenant:
(v) au moins un type de premier oligo-nucléotide choisi dans un groupe constitué par un premier oligonucléotide de type sauvage et d'un ou deux types d'un premier oligonucléotide qui a un site polymorphe de la séquence nucléotidique du chromosome ou fragment d'acide nucléique à l'extrémité 5' ;
(vi) un second oligonucléotide qui est complémentaire d'une chaîne de brin du chromosome ou fragment d'acide nucléique avec laquelle le premier oligonucléotide est hybridé et qui a une séquence en position adjacente au site en 5' du premier oligonucléotide et qui contient au moins une base sur l'extrémité 3' qui ne s'hybride pas avec le site polymorphe de la séquence nucléotidique ;
(vii) au moins un type d'enzyme possédant une activité nucléase ou une activité ligase ; et
(viii) une sonde de détection, laquelle peut détecter des produits issus de l'hybridation des premiers oligonucléotides et des seconds oligonucléotides obtenus en appliquant une activité ligase après avoir utilisé une activité nucléase pour déléter les séquences nucléotidiques qui ne forment pas des paires de bases au site polymorphe de la séquence nucléotidique telles que celles qui sont formées au niveau du site polymorphe de la séquence lorsque chaque oligo-nucléotide est hybridé.
